# EUROPÄISCHE PATENTSCHRIFT

(11) **EP 1 329 201 B1**
(45) Veröffentlichungstag und Bekanntmachung des Hinweises auf die Patenterteilung: **10.11.2004**
(21) Anmeldenummer: 02001415.5
(22) Anmeldetag: 21.01.2002
(51) Int. Cl.: A61B 17/28

(54) **Medizinisches Instrument für die manuell-unterstützte minimal invasive Chirurgie**
Medical instrument for hand assisted minimally invasive surgery
Instrument medical pour la chirurgie à caractère invasif minimum à assistance manuelle

(43) Veröffentlichungstag der Anmeldung: 23.07.2003
(73) Patentinhaber: Karl Storz GmbH & Co. KG, 78532 Tuttlingen (DE)
(72) Erfinder: Cuschieri, Sir Alfred, Prof., St. Andrews, Fife KY16 9TY (GB); Frank, Tim, Dr., Wormit, Fife (GB); Brown, Stuart, Dr., St. Andrews KY16 8QX (GB); Duncan, Martin, Dundee DD2 2EZ (GB); Gove, James, Dundee DD4 6LS (GB)
(74) Vertreter: Hofmeister, Frank Horst

(56) Entgegenhaltungen:
- EP-A- 0 788 775
- DE-A- 4 116 970
- US-A- 3 834 021
- US-A- 5 925 064
- US-A- 6 006 433

## Beschreibung

Die Erfindung betrifft ein medizinisches Instrument für die Chirurgie.

Obwohl die minimalinvasive Operationstechnik gegenüber der klassischen offenen Chirurgie unter anderem aufgrund der kürzeren Genesungszeiten der Patienten in den letzten Jahren verstärkt Anwendung findet, ist sie nicht dazu geeignet, die fene Chirurgie ganz zu verdrängen, da einerseits nicht alle Operationen auch endoskopisch durchführbar sind und andererseits der Operateur häufig das zu operierende Gewebe mit der Hand ergreifen und tastend untersuchen muß, was bei der endoskopischen Chirurgie nicht möglich ist.

Unter Laparoskopie versteht man die Inspektion der Bauchhöhle mit einem Laparoskop, das nach einer Stichinzision, gegebenenfalls nach einer Gasinsufflation beispielsweise mit CO₂, und Einstechen eines Trokars in die Bauchhöhle eingebracht wird. Bei der laparoskopischen Chirurgie werden unter Sichtkontrolle durch das Laparoskop mittels über dieselbe oder weitere Trokarhülsen in die Bauchhöhle eingeführter medizinischer Instrumente chirurgische Eingriffe in der Bauchhöhle durchgeführt.

Aus diesen unterschiedlichen Anforderungen und Möglichkeiten der beiden Operationstechniken, der minimalinvasiven Laparoskopie einerseits und der offenen Chirurgie andererseits, wurde vor etwa zwei Jahre die sogenannte HALS-Operationstechnik (Hand Assisted Laparoscopic Surgery) entwickelt, bei der zusätzlich zum Einbringen des Laparoskops und gegebenenfalls laparoskopischer Instrumente in die Bauchhöhle ein Hautschnitt für eine Hand des Operateurs geschaffen wird, damit dieser per Tastsinn und unter Beobachtung und Kontrolle durch das Laparoskop eine besser geführte Operation durchführen kann. Die HALS-Operationstechnik kombiniert somit die Vorteile der beiden bekannten Operationstechniken.

Um den Insufflationsdruck innerhalb der Bauchhöhle auch bei der HALS-Operationstechnik aufrechterhalten zu können, werden Manschetten verwendet, die über das Handgelenk des Operateurs gezogen werden und um den Einschnitt mit der Haut des Patienten verklebt werden. Neben der Druckbeständigkeit wird so eine sterile Abschottung des Operationsraums erreicht. Problematisch bei der HALS-Operationstechnik ist aber die Verwendung von chirurgischen Instrumenten, die mit der in die Bauchhöhle eingeführten Hand betätigt werden sollen, da diese mit der Hand in die Bauchhöhle eingebracht werden müssen. Neben der Gefahr, daß beim Einführen des Instruments Gewebe verletzt wird, ist ein Werkzeugwechsel aufgrund des möglichen Druckverlustes und der steigenden Infektionsgefahr nahezu ausgeschlossen.

Aus dem US-Patent 5 755 713 ist ein medizinisches Instrument für die laparoskopische Chirurgie bekannt, welches einen zylindrischen Schaft aufweist, in dem verschiedene Operationswerkzeuge gelagert sind. Über eine rechtwinklig vom Schaft abstehende Handhabe wird der Schaft in eine übliche Trokarhülse eingesetzt. Die innerhalb des Schaftes gelagerten Werkzeuge können von der Handhabe aus über ein Zahnradgetriebe angetrieben einzeln in eine Arbeitsposition außerhalb des Schaftes verfahren werden. Aufgrund der Anordnung mehrerer Werkzeuge in einem Instrumentenschaft ist dieses bekannte Instrument zwar für die laparoskopische Chirurgie vorteilhaft, aufgrund der Baugröße und der abgewinkelten Handhabe ist dieses bekannte medizinische Instrument aber auf keinen Fall für eine Operationstechnik, wie beispielsweise die HALS-Operationstechnik, geeignet, bei der das gesamte Instrument bei der Operation zusammen mit einer Hand des Operateurs in das Operationsgebiet eingeführt wird.

Aus der US-A-5925064 ist ein minimalinvasives Instrument zur laparoskopischen Chirurgie bekannt, das über ein Ringband an der Fingerspitze des Operateurs befestigt wird.

Davon ausgehend liegt der Erfindung die **Aufgabe** zugrunde, ein medizinisches Instrument für die Chirurgie zu schaffen, das insbesondere für die HALS-Operationstechnik geeignet ist.

Die **Lösung** dieser Aufgabenstellung ist ein medizinisches Instrument für die Chirurgie, das zu Operationszwecken zusammen mit einer Hand des Operateurs vollständig in das Operationsgebiet einführbar ist, und ein Gehäuse und mindestens ein in dem Gehäuse angeordnetes Werkzeug aufweist, das zwischen einer in dem Gehäuse angeordneten Ruhestellung und einer aus dem Gehäuse herausragenden Arbeitsstellung verlagerbar ist.

Durch die erfindungsgemäße Ausbildung des Instruments, bei dem das chirurgische Werkzeug zwischen einer Ruhestellung und einer Arbeitsstellung verlagerbar ist, wird die Verletzungsgefahr beim Einführen des Werkzeugs für den Patienten vollständig beseitigt, da der Operateur das Werkzeug erst im Operationsgebiet in die Arbeitsstellung überführen muß. Beim Herausziehen der Hand aus der Bauchhöhle verlagert der Operateur dann das Werkzeug wieder zurück in die gesicherte Ruhestellung im Instrumentengehäuse. Ein solchermaßen ausgestaltetes medizinisches Instrument ist insbesondere für die HALS-Operationstechnik bestens geeignet.

Um auch das Problem des Werkzeugwechsels während der Operation zu lösen, wird gemäß einer bevorzugten Ausführungsform der Erfindung vorgeschlagen, daß mehrere Werkzeuge auswechselbar und zwischen der Ruhestellung und der Arbeitsstellung verlagerbar in dem Gehäuse angeordnet sind. Somit entfällt für den Operateur der externe Wechsel der chirurgischen Werkzeuge. Zum Wechseln muß der Operateur lediglich das zuletzt benutzte Werkzeug wieder zurück in die Ruhestellung im Gehäuse verlagern und ein neues Werkzeug in die Arbeitsstellung überführen.

Das eigentliche Verlagern der Werkzeuge zwischen der Ruhestellung und der Arbeitsstellung kann translatorisch oder rotatorisch erfolgen, wobei aber die rein translatorische Bewegung zu bevorzugen ist, da diese Bewegung den geringsten Raum beansprucht und die Verletzungsgefahr für den Patienten minimiert.

Die Bediensicherheit des erfindungsgemäßen Instruments läßt sich weiter dadurch erhöhen, daß immer nur ein einzelnes Werkzeug aus dem Gehäuse heraus in die Arbeitsstellung verlagerbar ist, so daß nicht versehentlich zwei oder mehr Werkzeuge gleichzeitig in die Arbeitsstellung überführt werden. Neben der Ausbildung einer ausreichend kleinen Werkzeugaustrittsöffnung im Gehäuse kann diese Sicherung gegen das Verlagern mehrerer Werkzeuge erfindungsgemäß dadurch erzielt werden, daß das in die Arbeitsstellung verlagerte Werkzeug automatisch die anderen Werkzeuge innerhalb des Gehäuses in der Ruhestellung fixiert.

Weiterhin wird mit der Erfindung vorgeschlagen, daß das in die Arbeitsstellung verlagerte Werkzeug in der Arbeitsstellung ortsfest fixiert ist, um dem Operateur ein exaktes Führen des Werkzeugs zu ermöglichen.

Um zu verhindern, daß der Operateur das in das Operationsgebiet eingeführte medizinische Instrument im Operationsgebiet verliert, ist das Gehäuse mit der Hand des Operateurs verbunden, insbesondere ist am Gehäuse ein Fingerring zum verliersicheren Festlegen an der Hand des Operateurs angeordnet. Gemäß einer bevorzugten Ausführungsform der Erfindung ist der Fingerring über einen verschwenkbar am Gehäuse gelagerten Steg mit dem Gehäuse verbunden. Durch die Verwendung dieses Stegs läßt sich die Einsatzlänge des Instruments im Operationsgebiet verlängern.

Weiterhin wird mit der Erfindung vorgeschlagen, daß, wenn mindestens ein Werkzeug ein mit zwei gegeneinander verstellbaren Maulteilen versehenes Greif- oder Schneidwerkzeug, insbesondere ein Nadelgreifer, ist, dieses Werkzeug zum Fixieren der Maulteile in der geschlossenen Stellung, teilweise in das Gehäuse einziehbar ist. Auf diese Weise besteht die Möglichkeit, daß das Greifwerkzeug weiter einen Gegenstand, wie beispielsweise eine Nadel, festhält und der Operateur gleichzeitig die Hand zumindest teilweise wieder frei hat, um beispielsweise einen Faden zu ergreifen. Die beiden gegeneinander verstellbaren Maulteile des Greif- oder Schneidwerkzeugs sind über ein Federelement in Öffnungsrichtung der Maulteile vorgespannt.

Das Gehäuse des erfindungsgemäßen medizinischen Instruments besteht vorteilhafterweise aus zwei im wesentlichen parallel zueinander angeordneten Wänden, die über ein zwischen den Wänden angeordnetes Basisteil voneinander beabstandet sind, wobei das Basisteil gemäß einer bevorzugten Ausführungsform T-förmig ausgebildet ist und die Werkzeuge zwischen den Wänden und dem senkrechten Schenkel des T-förmigen Basisteils gelagert sind. Bei dieser Ausgestaltung ergeben sich automatisch zwischen den Innenseiten der Wände und dem senkrechten Schenkel des Basisteils Führungsschächte für die im Gehäuse zu lagernden Werkzeuge.

Damit verhindert wird, daß eines der auswechselbar im Gehäuse angeordneten Werkzeuge sich während der Operation lösen kann, ist das mindestens eine Werkzeug nur mittels Zweihandbedienung aus dem Gehäuse entnehmbar. Zu diesem Zweck ist ein zwischen wenigstens einer Wand des Gehäuses sowie einem Werkzeug zusammenwirkender Entriegelungsmechanismus vorgesehen, der bei einer praktischen Ausführungsform der Erfindung aus einem in einer Wand gelagerten Entriegelungsknopf besteht.

Der vorteilhafterweise federnde Lagerung des Entriegelungsknopfs wird gemäß einer praktischen Ausführungsform der Erfindung dadurch erzielt, daß der Entriegelungsknopf auf einer in einer Ausnehmung der Wand gelagerten, freigeschnittenen Federzunge angeordnet ist.

Zum Führen und Halten des Werkzeugs innerhalb des Gehäuses weist der Entriegelungsknopf einen Führungsstift auf der in eine Führungsbahn des entsprechenden Werkzeugs eingreift. Die Ausfahrlänge des mindestens einen Werkzeugs aus dem Gehäuse ist durch einen Anschlag begrenzt, um eine definierte und immer gleiche Länge des Werkzeugs zu gewährleisten. Vorteilhafterweise ist der Anschlag gleichzeitig ein Teil des Verriegelungsmechanismus, insbesondere der Führungsbahn für den Führungsstift des Entriegelungsknopfs.

Um sicherzustellen, daß gleichzeitig immer nur ein Werkzeug in die Arbeitsstellung verschiebbar ist, ist ein zwischen dem Basisteil des Gehäuses sowie einem Werkzeug zusammenwirkender Kopplungsmechanismus vorgesehen, der aus einem im Basisteil gelagerten Federelement besteht. Zur Erzielung der Kopplung sind in den Werkzeugen Ausnehmungen derart ausgebildet sind, daß das im Basisteil gelagerte Federelement in der Ruhestellung der Werkzeuge in die Ausnehmungen zweier parallel nebeneinander im Gehäuse angeordneter Werkzeuge eingreift, wobei pro parallel im Gehäuse angeordnetem Werkzeugpaar ein Federelement vorgesehen ist. Die Bediensicherheit kann weiterhin dadurch verbessert werden, daß mehrere im Gehäuse angeordnete Federelemente so miteinander gekoppelt verbunden sind, daß das Verlagern eines Federelements aus der Ruhestellung die jeweils anderen Federelemente blockiert.

Damit die Werkzeuge in der Arbeitsstellung so arretierbar sind, daß sie nicht bei einer axialen Schubbelastung sofort wieder in das Gehäuse hineingeschoben werden, sind in den Werkzeugen zusätzliche Ausnehmungen ausgebildet, in die das im Basisteil gelagerte Federelement in der Arbeitsstellung des jeweiligen Werkzeugs eingreift.

Gemäß einer weiteren praktischen Ausführungsform der Erfindung wird vorgeschlagen, daß am Basisteil jeweils wenigstens ein in einen jeden Führungsschacht hineinragender Vorsprung ausgebildet ist, der in eine entsprechende Vertiefung im jeweiligen Werkzeug eingreift. Diese Kopplung verhindert, daß das Werkzeug geöffnet werden kann, bevor es vollständig in die Arbeitsstellung verschoben wurde. Zusätzlich wird vorgeschlagen, daß am proximalen Ende der Vertiefung eine Anlaufschräge ausgebildet ist. Gegen diese Anlaufschräge läuft der jeweilige Vorsprung des Werkzeugs an, wenn das Werkzeug in das Gehäuse zurückgeschoben wird. Dabei kann das Werkzeug in einer geringfügig geöffneten Position gehalten werden, ohne daß der Operateur das Werkzeug, beispielsweise einen Nadelhalter, selbst ergreifen muß. Das Werkzeug wird eingeschoben, so daß der Vorsprung auf der Anlaufschräge eine dem Öffnungsgrad des werkzeugs entsprechende Strecke aufläuft. Dabei muß die Neigung der Anlaufschräge so gestaltet sein, daß der Vorsprung bei allen klemmbaren Werkzeugöffnungsgraden selbsthemmend gehalten wird.

Weiterhin wird mit der Erfindung vorgeschlagen, daß die Oberfläche des Gehäuses glatt und frei von scharfen Kanten ausgebildet ist, damit das Instrument völlig gefahrlos mit der Hand des Operateurs in das Operationsgebiet eingeführt werden kann.

Schließlich wird mit der Erfindung vorgeschlagen, daß am Gehäuse ein Lichtleiter und/oder ein flexibles Endoskop führend gelagert sind, um so die Flexibilität des Instruments weiter zu erhöhen und dieses auch als eigenständiges chirurgisches Operationsinstrument verwenden zu können.

Weitere Merkmale und Vorteile der Erfindung ergeben sich anhand der nachfolgenden Beschreibung der zugehörigen Zeichnung, in der zwei Ausführungsbeispiele eines erfindungsgemäßen medizinischen Instruments nur beispielhaft schematisch dargestellt sind. In der Zeichnung zeigt:
- Fig. 1: eine perspektivische Ansicht einer ersten Ausführungsform eines erfindungsgemäßen medizinischen Instruments in der Arbeitsstellung;
- Fig. 2: eine perspektivische Ansicht einer zweiten Ausführungsform eines erfindungsgemäßen medizinischen Instruments in der Arbeitsstellung;
- Fig. 3a: eine schematische Seitenansicht des medizinischen Instruments gemäß Fig. 1 in der Ruhestellung;
- Fig. 3b: eine schematische Draufsicht auf das Instrument gemäß Fig. 3a;
- Fig. 4: eine schematische perspektivische Ansicht des Basisteils des Instruments gemäß Fig. 3a und 3b;
- Fig. 5: eine schematische Rückansicht des Instrumentengehäuses gemäß Fig. 3a und 3b, jedoch ohne in dem Gehäuse angeordneten Werkzeugen;
- Fig. 6a: eine schematische perspektivische Ansicht einer Wand des Instruments gemäß Fig. 3a und 3b;
- Fig. 6b: einen Schnitt entlang der Schnittlinie Vlb-Vlb gemäß Fig. 6a;
- Fig. 7: eine Seitenansicht eines Greifwerkzeugs und
- Fig. 8: eine Seitenansicht eines Schneidwerkzeugs.

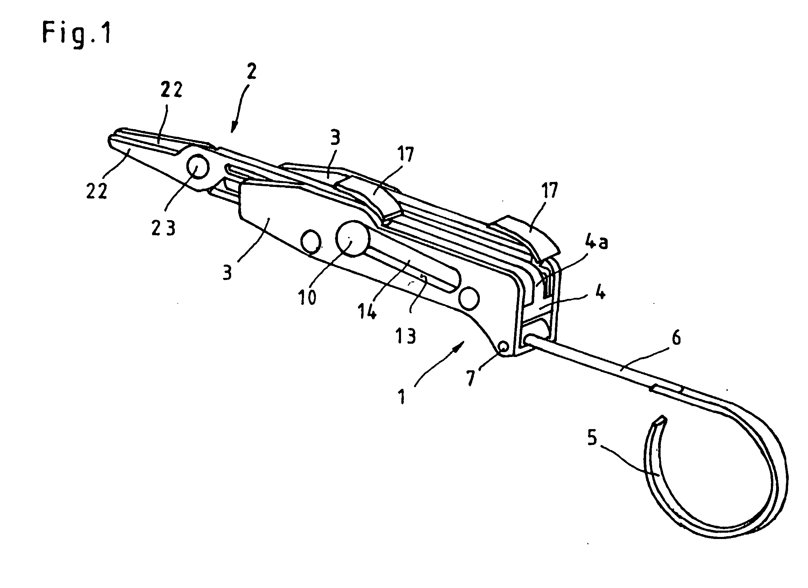

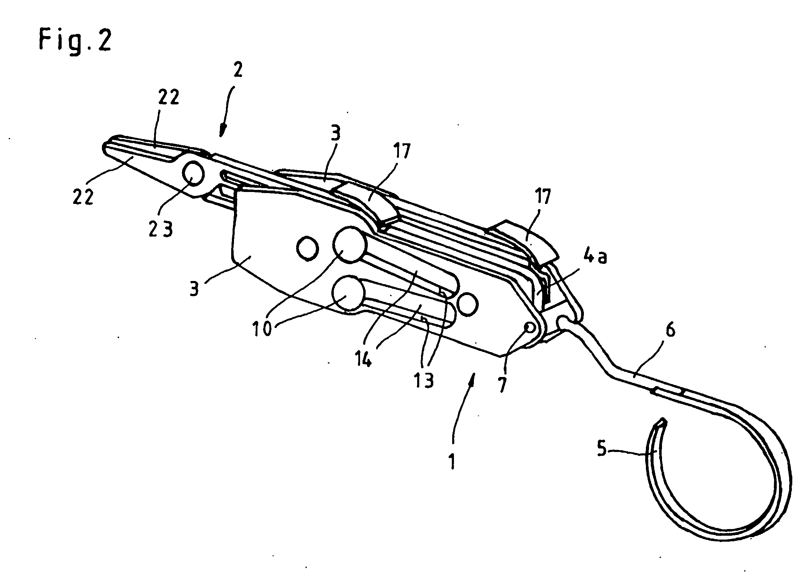

Bei den in den Abbildungen Fig. 1 und 2 in perspektivischer Ansicht dargestellten medizinischen Instrumenten handelt es sich um chirurgische Instrumente, die insbesondere für die laparoskopische Chirurgie mittels der HALS (Hand Assisted Laparoscopic Surgery) Operationstechnik geeignet sind, bei der zusätzlich zur Verwendung eines Laparoskops und gegebenenfalls der Verwendung bekannter, durch Trokarhülsen in die Bauchhöhle einführbarer Instrumente, ein Hautschnitt vorgenommen wird, durch den der Operateur eine Hand in die Bauchhöhle einführt, um dort unter Beobachtung und Kontrolle durch das Laparoskop und mittels seines Tastsinns die Operation zu unterstützen bzw. durchzuführen.

Diese dargestellten Instrumente werden zusammen mit der Hand des Operateurs vollständig in das Operationsgebiet, die Bauchhöhle, eingeführt und dort mittels der eingeführten Hand bedient.

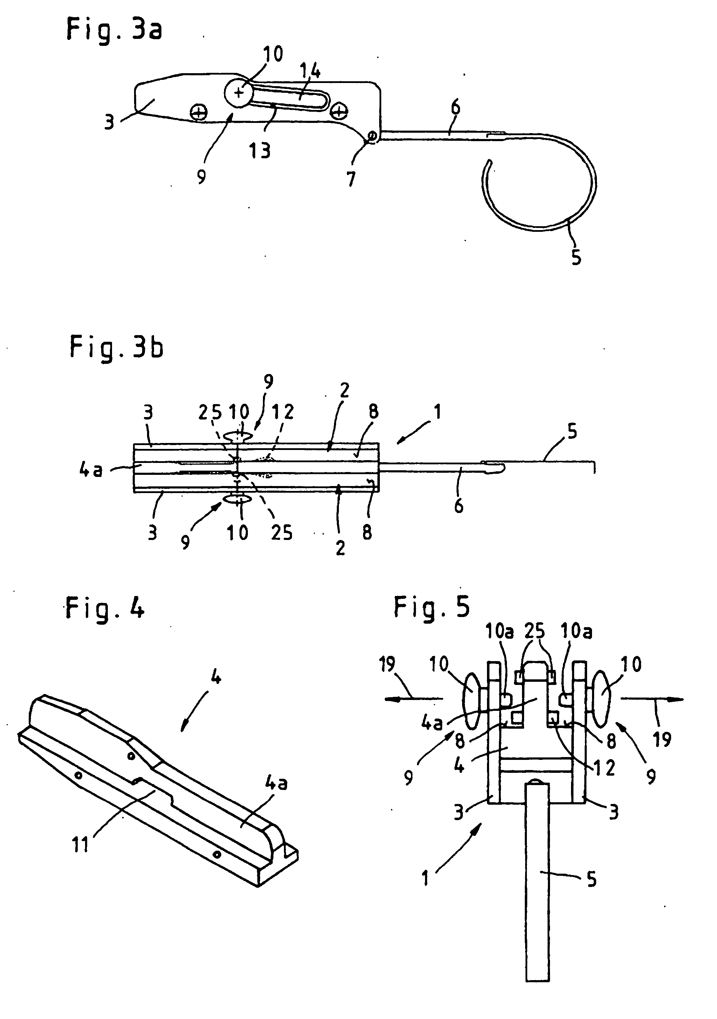

Wie insbesondere aus den Abbildungen Fig. 3a bis Fig. 6b ersichtlich, besteht ein solches medizinisches Instrument im wesentlichen aus einem Gehäuse 1, in dem mindestens ein chirurgisches Werkzeug 2 derart gelagert ist, daß dieses mindestens eine Werkzeug 2 zwischen einer vollständig in das Gehäuse 1 eingefahrenen Ruhestellung (Fig. 3a und 3b) und einer aus dem Gehäuse 1 herausragenden Arbeitsstellung (Fig. 1 und 2) verlagerbar ist.

Das Gehäuse 1 besteht aus zwei parallel zueinander angeordneten Wänden 3 und einem zwischen den Wänden 3 angeordneten Basisteil 4, die bei dem dargestellten Ausführungsbeispiel miteinander verschraubt sind. Das dargestellte Basisteil 4 ist, wie aus Fig. 4 ersichtlich, im wesentlichen T-förmig ausgebildet so zwischen den beiden Wänden 3 angeordnet, daß der senkrechte Schenkel 4a des T-förmigen Basisteils 4 parallel zwischen den beiden Wänden 3 liegt.

Damit der Operateur dieses medizinische Instrument verliersicher während der Operation bedienen kann, ist das Gehäuse 1 mit einem Fingerring 5 versehen, der über einen Steg 6 mit dem Gehäuse 1 verbunden ist. Bei der in den Abbildungen dargestellten bevorzugten Ausführungsform ist der Steg 6 um eine Schwenkachse 7 verschwenkbar am Gehäuse 1 gelagert, so daß das medizinische Instrument ausgehend von den Darstellungen gemäß Fig. 1 und 2 auf den Fingerring 5 zu zusammengeklappt werden kann. In dieser zusammengeklappten Position findet das medizinische Instrument Aufnahme in der Handfläche des Operateurs und läßt sich auf diese Weise zusammen mit der Hand des Operateurs einfach und sicher durch den Hauteinschnitt in die Bauchhöhle des Patienten einführen.

Die Verwendung des um die Schwenkachse 7 verschwenkbaren Fingerrings 5 ermöglicht die Verwendung eines in der ausgeklappten Arbeitsposition relativ großen Instruments. Selbstverständlich ist es auch möglich, den Fingerring 5 über den Steg 6 starr mit dem Gehäuse 1 zu verbinden. Bei dieser Ausführungsform ist die Gesamtlänge des Instruments geringer als bei der zuvor beschriebenen Variante, damit das medizinische Instrument zum Einführen in das Operationsgebiet Aufnahme in der Handfläche des Operateurs finden kann.

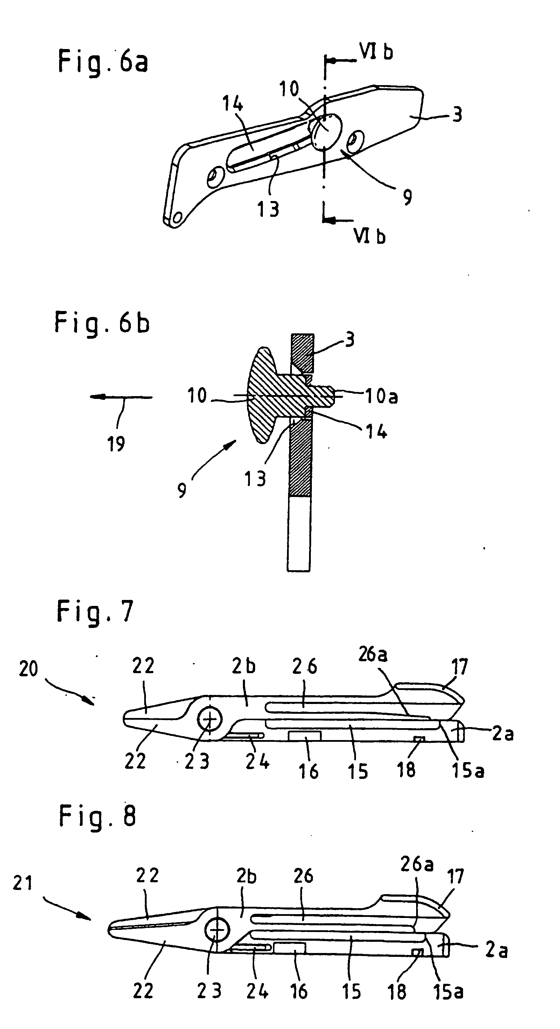

Wie aus der Draufsicht gemäß Fig. 3b sowie der Rückansicht gemäß Fig. 5 ersichtlich, werden durch den Aufbau des Gehäuses 1, mit den drei parallel zueinander angeordneten Stegen, nämlich den beiden Wänden 3 und dem senkrechten Schenkel 4a des Basisteils 4, zwei Führungsschächte 8 zur Aufnahme und zum Führen der Werkzeuge 2 geschaffen. Die Werkzeuge 2 sind auswechselbar in dem Gehäuse 1 angeordnet, so daß der Operateur vor der Operation individuell für die jeweilige Operation das medizinische Instrument mit Werkzeugen 2 bestücken kann, die er für die nachfolgende Operation benötigt. Die Führungsschächte 8 und Werkzeuge 2 sind dabei so ausgebildet, daß jedes Werkzeug 2 in jeden Führungsschacht 8 einsetzbar ist, so daß Fehlbelegungen ausgeschlossen sind.

Um sicherzustellen, daß die Werkzeuge 2 sicher und fest im Gehäuse 1 gelagert sind, ist ein Entriegelungsmechnismus 9 vorgesehen, der nur mit zwei Händen zu betätigen ist, so daß ein versehentliches Entriegeln der Werkzeuge 2 während der Operation unmöglich ist. Weiterhin ist ein Kopplungsmechanismus vorgesehen, der dazu dient, die einzelnen in dem Gehäuse 1 gelagerten Werkzeuge 2 so miteinander zu koppeln, daß das gleichzeitige Ausfahren von mehr als einem Werkzeug 2 verhindert wird.

Während der dargestellte, und insbesondere aus den Abbildungen Fig. 3a bis 8 ersichtliche Entriegelungsmechanismus 9 im wesentlichen aus jeweils einem in einer Wand 3 federnd gelagerten Entriegelungsknopf 10 mit Führungsstift 10a pro Werkzeug 2 besteht, wird der Kopplungsmechanismus von einem in einer Ausnehmung 11 des Basisteils 4 gelagerten Federelement 12 gebildet.

Die Entriegelungsknöpfe 10 sind in Ausnehmungen 13 der jeweiligen Wände 3 federnd gelagert, wobei die federnde Lagerung bei den dargestellten Ausführungsformen durch jeweils eine in den Ausnehmungen 13 gelagerte, freigeschnittene Federzunge 14 bewirkt wird. Zur Aufnahme des Führungsstifts 10a des jeweiligen Entriegelungsknopfs 10 ist in den unteren Teilen 2a der Werkzeuge 2 jeweils eine im wesentlichen parallel zur Längsachse des Werkzeugs 2 verlaufende Führungsbahn 15 ausgebildet. Weiterhin sind in den Werkzeugen 2 Ausnehmungen 16 zur Aufnahme des Federelements 12 ausgebildet, in die das Federelement 12 in der Ruhestellung der jeweiligen Werkzeuge 2 fixierend eingreift.

Wird nun ein Werkzeug 2 aus der in Fig. 3a und 3b dargestellten Ruhestellung über einen Griffbereich 17 nach vorne in die Arbeitsstellung gemäß Fig. 1 und 2 geschoben, wird das Federelement 12 aus der Ausnehmung 16 dieses Werkzeugs 2 heraus und tiefer in die Ausnehmung 16 des daneben angeordneten Werkzeugs 2 gedrückt, so daß dieses zweite Werkzeug 2 in der Ruhestellung fixiert ist und nicht gleichzeitig in die Arbeitsstellung verschoben werden kann.

Bei der Verwendung eines medizinischen Instruments mit vier in dem Gehäuse 1 angeordneten Werkzeugen 2, wie dies der Abbildung Fig. 2 zu entnehmen ist, ist ein Federelement 12 pro Werkzeugpaar vorgesehen, wobei die beiden Federelemente 12 so miteinander gekoppelt sein können, daß beim Ausschieben eines Werkzeugs 2 in die Arbeitsstellung auch das Federelement 12 des anderen Werkzeugpaars blockiert ist und das Ausschieben eines weiteren Werkzeugs 2 verhindert.

Ergänzend zur Ausnehmung 16 zur Aufnahme des Federelements 12 in der Ruhestellung des jeweiligen Werkzeugs 2 kann in jedem Werkzeug 2 eine weitere Ausnehmung 18 ausgebildet sein, in die das Federelement 12 in der Arbeitsstellung des Werkzeugs 2 eingreift, um das Werkzeug in der Arbeitsstellung zu arretieren und ein versehentliches Einschieben des Werkzeugs 2 in das Gehäuse 1 zu verhindern. Um aber zu verhindern, daß aufgrund der zweiten Aufnahme 18 die gegenseitige Blockierung der Werkzeuge 2 aufgehoben wird, die sicherstellt, daß gleichzeitig immer nur ein Werkzeug 2 in die Arbeitsstellung verschoben werden kann, muß sich die zweite Ausnehmung 18 von der ersten Ausnehmung 16 in Form und/oder Tiefe derart unterscheiden, daß die Hauptfederkraft des Federelements 12 auf die Ausnehmung 16 des zu blockierenden Werkzeugs 2 gerichtet ist.

Zusätzlich zum Arretieren des Werkzeugs 2 in der Arbeitsstellung über das Eingreifen des Federelements 12 in die Ausnehmung 18 wird der Verschiebeweg des Werkzeugs 2 in Richtung der Arbeitsstellung durch einen das Ende der Führungsbahn 15 bildenden Anschlag 15a begrenzt, gegen den beim Ausschieben des Werkzeugs 2 in die Arbeitsstellung der in der Führungsbahn 15 gelagerte Führungsstift 10a des Entriegelungsknopfs 10 anläuft.

Der Anschlag 15a der Führungsbahn 15 verhindert somit auch, daß das Werkzeug 2 beim Verschieben in die Arbeitsstellung versehentlich ganz aus dem Gehäuse 1 entfernt wird und so verloren gehen kann. Zum Entriegeln und Auswechseln eines Werkzeugs 2 ist es somit notwendig, den Führungsstift 10a des jeweiligen Entriegelungsknopfs 10 außer Eingriff mit der Führungsbahn 15 zu bringen. Zu diesem Zweck ist der Entriegelungsknopf 10 federnd in der Wand 3 des Gehäuses 1 gelagert. Soll nun ein Werkzeug 2 ausgewechselt werden wird der Entriegelungsknopf 10 in Richtung des Pfeils 19 in Fig.5 senkrecht zur Wand 3 nach außen herausgezogen, bis der Führungsstift 10a nicht mehr in die Führungsbahn 15 eingreift. In dieser Stellung kann nun das entsprechende Werkzeug 2 über den Griffbereich 17 aus dem Gehäuse 1 herausgeschoben werden. Da das gleichzeitige Herausziehen des Entriegelungsknopfs 10 und das Herausschieben des Werkzeugs 2 nur mit zwei Händen erfolgen kann, wird automatisch ein unbeabsichtigtes Entriegeln des Werkzeugs 2 während der Operation im Operationsgebiet verhindert.

Die beiden Abbildungen gemäß Fig. 1 und 2 unterscheiden sich dadurch voneinander, daß bei dem medizinischen Instrument gemäß Fig. 1 zwei Werkzeuge 2 und bei dem medizinischen Instrument gemäß Fig. 2 vier Werkzeuge 2 in dem Gehäuse 1 angeordnet sind. Die Anordnung der vier Werkzeuge 2 ist daran zu erkennen, daß in der Wand 3 zwei Ausnehmungen 13 für zwei Entriegelungsknöpfe 10 ausgebildet sind. Die Werkzeuge 2 sind paarweise übereinander im Gehäuse 1 angeordnet.

In den Abbildungen Fig. 7 und 8 sind beispielhaft zwei Werkzeuge 2 dargestellt, nämlich ein Greifwerkzeug 20 (Fig. 7) und ein Schneidwerkzeug 21 (Fig. 8). Beide Werkzeuge 20 und 21 weisen jeweils zwei gegeneinander verstellbare Maulteile 22 auf, die um eine Schwenkachse 23 gegeneinander zwischen einer geöffneten und einer geschlossenen Stellung verstellbar sind. Über ein im Bereich der Schwenkachse 23 angeordnetes Federelement 24 sind die Maulteile 22 in die geöffnete Stellung vorgespannt, so daß sich die Maulteile 22 automatisch öffnen, wenn sich das Werkzeug 20, 21 in der vollständig ausgefahrenen Arbeitsstellung befindet.

Wie aus Fig. 1 und 2 ersichtlich, besteht die Möglichkeit, die Maulteile 22 in der geschlossenen Stellung dadurch zu fixieren, daß das Werkzeug 20, 21 teilweise zurück in das Gehäuse 1 geschoben wird. Dies wird dadurch erreicht, daß, wie aus den Abbildungen Fig. 3b und 5 ersichtlich, am senkrechten Schenkel 4a des Basisteils 4 beidseitig nach außen vorstehende, in die Führungsschächte 8 hineinragende Vorsprünge 25 ausgebildet sind, wobei jeder Vorsprung 25 in eine entsprechende Vertiefung 26 im oberen Teil 2b eines jeden Werkzeugs 2 eingreift, wie diese den Abbildungen Fig. 7 und 8 zu entnehmen ist.

Durch den Eingriff des Vorsprungs 25 in die Vertiefung 26 kann der obere Teil 2b des Werkzeugs 2 nicht nach oben verschwenkt werden, bis das Werkzeug 2 vollständig nach vorne in die Arbeitsposition verschoben wurde und der Vorsprung aus dem offenen proximalen Ende der Vertiefung 26 heraustritt.

Wie weiterhin aus Fig. 7 und 8 ersichtlich weist die Vertiefung 26 am proximalen Ende eine Anlaufschräge 26a auf. Wird nun das Werkzeug 2 aus der Arbeitsstellung wieder leicht in das Gehäuse 1 zurückgedrückt, läuft der Vorsprung 25 die durch die Anlaufschräge 26a gebildete Rampe herauf und schließt automatisch die beiden Maulteile 22 des Werkzeugs 20, 21. In dieser Stellung besteht beispielsweise die Möglichkeit, daß das Greifwerkzeug 20 einen Gegenstand, wie beispielsweise eine Nadel festhält, während der Operateur mit der im Operationsgebiet befindlichen Hand eine andere Tätigkeit durchführt, da das Werkzeug 2 aufgrund des Eingriffs des Vorsprungs 25 in die Vertiefung 26 wieder gegen ein weiteres Öffnen gesperrt ist.

Um sicherzustellen, daß jedes Werkzeug 2 in jeden Führungsschacht 8 eingesetzt werden kann, sind die Führungsbahn 15 für den Führungsstift 10a des Entriegelungsknopfs 10 sowie die Vertiefung 26 zur Aufnahme des Vorsprungs 25 entweder als durchgehende Einschnitte im unteren Teil 2a bzw. oberen Teil 2b des Werkzeugs 2 oder aber als beidseitig in den Werkzeugteilen 2a, 2b ausgebildete Einfräsungen ausgebildet.

Das Arbeiten mit dem in den Abbildungen Fig. 1 bis 8 dargestellten medizinischen Instrument geschieht wie folgt:

Vor der laparoskopischen Operation mittels der HALS-Operationstechnik wählt der Operateur die Werkzeuge 2 aus, die er für die durchzuführende Operation benötigt. Diese Werkzeuge 2 werden nach Betätigen des Entriegelungsknopfes 10 in das Gehäuse 1 eingesetzt und verrasten sich dort in dem Entriegelungsmechanismus 9.

Um das medizinische Instrument zusammen mit der Hand des Operateurs in die Bauchhöhle des Patienten einzuführen, werden alle Werkzeuge 2 in die Ruhestellung im Gehäuse 1 verlagert und das Gehäuse 1 über die Schwenkachse 7 so auf den Fingerring 5 zu zusammengeklappt, daß das medizinische Instrument Aufnahme in der Handfläche des Operateurs findet. Jetzt kann der Operateur seine Hand mitsamt dem über den Fingerring 5 unverlierbar an der Hand des Operateurs festgelegten medizinischen Instrument durch einen geeigneten Hautschnitt in die Bauchhöhle des Patienten einbringen.

Zur Bedienung des medizinischen Instruments muß der Operateur jetzt zunächst das medizinische Instrument wieder um die Schwenkachse 7 ausklappen, bis es bedienbereit in seiner Hand liegt. Das Ausfahren eines Werkzeugs 2 erfolgt mittels des Daumens des Operateurs, mit dem er über den Griffbereich 17 des jeweiligen Werkzeugs 2 das Werkzeug 2 aus dem Gehäuse 1 heraus in die Arbeitsstellung schiebt. In dieser Arbeitsstellung ist das Werkzeug 2 lagesicher fixiert, damit der Operateur das Werkzeug exakt führen kann.

Benötigt der Operateur ein anderes Werkzeug 2, so muß er zunächst das zuvor benutzte Werkzeug 2 zurück in die Ruhestellung im Gehäuse 1 verlagern, da sich immer nur ein Werkzeug 2 zur gleichen Zeit in der Arbeitsstellung befinden kann und darf. Nach Beendigung der Operation verschiebt der Operateur alle Werkzeuge 2 in die Ruhestellung und klappt das medizinische Instrument wieder zusammen, so daß er es in der geschlossenen Handfläche aus der Bauchhöhle des Patienten entfernen kann.

Zu Reinigungszwecken, insbesondere zum Autoklavieren, läßt sich das medizinische Instrument einfach und schnell vollständig zerlegen.

Obwohl bei den dargestellten Ausführungsbeispielen das Verlagern der Werkzeuge 2 zwischen der Ruhestellung und der Arbeitsstellung über eine rein translatorische Bewegung erfolgt, ist es selbstverständlich auch möglich, diese Bewegung rotatorisch oder kombiniert rotatorisch und translatorisch zu vollziehen. Ebenso besteht durchaus die Möglichkeit, mehr als nur vier Werkzeuge 2 in dem Gehäuse 1 anzuordnen.

Insgesamt zeichnen sich die dargestellten medizinischen Instrumente dadurch aus, daß sie sehr kompakt und vielseitig verwendbar aufgebaut sind, so daß sie dazu geeignet sind, die bestehenden Probleme bei der HALS-Operationstechnik, insbesondere das des Werkzeugwechsels während der Operation, vollständig zu lösen.

Zusätzlich zu den dargestellten Ausführungsbeispielen besteht die Möglichkeit, das medizinische Instrument mit einem am Gehäuse 1 gelagerten Lichtleiter und/oder flexiblen Endoskop auszustatten. Ein solchermaßen ausgestattetes medizinische Instrument ist insbesondere dazu geeignet, nicht nur eine laparoskopische Operation nach Art und Weise der HALS-Operationstechnik zu unterstützen, sondern für eigenständige Operationen, beispielsweise in der Bauchhöhle, verwendet zu werden.

Bei der Verwendung des mit einem Lichtleiter und/oder einem flexiblen Endoskop versehenen Instruments können die Zuleitungen zum Lichtleiter und/oder zum Endoskop so gelegt werden, daß am Arm des Operateurs entlang verlaufen und entweder innerhalb oder außerhalb des Handschuhs des Operateurs verlaufen. In beiden Fällen ist für eine sterile und gasdichte Abdichtung der Zuleitungen an der das Operationsgebiet auf der Körperaußenseite umgebenden Manschette und eventuell zusätzlich am Übergang in den Handschuh des Operateurs zu sorgen.

### Bezugszeichenliste

- 1: Gehäuse
- 2: Werkzeug
- 2a: unterer Teil
- 2b: oberer Teil
- 3: Wand
- 4: Basisteil
- 4a: senkrechter Schenkel
- 5: Fingerring
- 6: Steg
- 7: Schwenkachse
- 8: Führungsschacht
- 9: Entriegelungsmechnismus
- 10: Entriegelungsknopf
- 10a: Führungsstift
- 11: Ausnehmung
- 12: Federelement
- 13: Ausnehmung
- 14: Federzunge
- 15: Führungsbahn
- 15a: Anschlag
- 16: Ausnehmung
- 17: Griffbereich
- 18: Ausnehmung
- 19: Pfeil
- 20: Greifwerkzeug
- 21: Schneidwerkzeug
- 22: Maulteil
- 23: Schwenkachse
- 24: Federelement
- 25: Vorsprung
- 26: Vertiefung
- 26a: Anlaufschräge

## Patentansprüche

1. Medizinisches Instrument für die Chirurgie, das zu Operationszwecken zusammen mit einer Hand des Operateurs vollständig in das Operationsgebiet einführbar ist, und ein Gehäuse (1) und mindestens ein in dem Gehäuse (1) angeordnetes Werkzeug (2) aufweist, das zwischen einer in dem Gehäuse (1) angeordneten Ruhestellung und einer aus dem Gehäuse (1) herausragenden Arbeitsstellung verlagerbar ist, wobei mindestens ein Werkzeug (2) ein mit zwei gegeneinander verstellbaren Maulteilen (22) versehenes Greifoder Schneidwerkzeug (20, 21), insbesondere ein Nadelgreifer, ist und am Gehäuse (1) ein Fingerring (5) zum Festlegen an der Hand des Operateurs angeordnet ist, wobei
das Werkzeug (20, 21) zum Fixieren der Maulteile (22) in der geschlossenen Stellung, teilweise in das Gehäuse (1) einziehbar ist **dadurch gekennzeichnet, daß** der Fingerring (5) über einen verschwenkbar am Gehäuse (1) gelagerten Steg (6) mit dem Gehäuse (1) verbunden ist.

2. Medizinisches Instrument nach Anspruch 1, **dadurch gekennzeichnet, daß** mehrere Werkzeuge (2) auswechselbar und zwischen der Ruhestellung und der Arbeitsstellung verlagerbar in dem Gehäuse (1) angeordnet sind.

3. Medizinisches Instrument nach Anspruch 1 oder 2, **dadurch gekennzeichnet, daß** das Verlagern des mindestens einen Werkzeugs (2) zwischen der Ruhestellung und der Arbeitsstellung über eine translatorische Bewegung erfolgt.

4. Medizinisches Instrument nach Anspruch 1 oder 2, **dadurch gekennzeichnet, daß** das Verlagern des mindestens einen Werkzeugs (2) zwischen der Ruhestellung und der Arbeitsstellung über eine rotatorische Bewegung erfolgt.

5. Medizinisches Instrument nach mindestens einem der Ansprüche 2 bis 4, **dadurch gekennzeichnet, daß** immer nur ein einzelnes Werkzeug (2) aus dem Gehäuse (1) heraus in die Arbeitsstellung verlagerbar ist.

6. Medizinisches Instrument nach Anspruch 5, **dadurch gekennzeichnet, daß** das in die Arbeitsstellung verlagerte Werkzeug (2) die anderen Werkzeuge (2) innerhalb des Gehäuses (1) in der Ruhestellung fixiert.

7. Medizinisches Instrument nach mindestens einem der Ansprüche 1 bis 6, **dadurch gekennzeichnet, daß** das in die Arbeitsstellung verlagerte Werkzeug (2) in der Arbeitsstellung ortsfest fixiert ist.

8. Medizinisches Instrument nach mindestens einem der Ansprüche 1 bis 7, **dadurch gekennzeichnet, daß** die beiden gegeneinander verstellbaren Maulteile (22) über ein Federelement (24) in Öffnungsrichtung der Maulteile (22) vorgespannt sind.

9. Medizinisches Instrument nach mindestens einem der Ansprüche 1 bis 8, **dadurch gekennzeichnet, daß** das Gehäuse (1) aus zwei im wesentlichen parallel zueinander angeordneten Wänden (3) besteht, die über ein zwischen den Wänden (3) angeordnetes Basisteil (4) voneinander beabstandet sind.

10. Medizinisches Instrument nach Anspruch 9, **dadurch gekennzeichnet, daß** das Basisteil (4) im wesentlichen T-förmig ausgebildet ist.

11. Medizinisches Instrument nach Anspruch 10, **dadurch gekennzeichnet, daß** die Werkzeuge (2) zwischen den Wänden (3) und dem senkrechten Schenkel (4a) des T-förmigen Basisteils (4) gelagert sind.

12. Medizinisches Instrument nach mindestens einem der Ansprüche 1 bis 11, **dadurch gekennzeichnet, daß** das mindestens eine Werkzeug (2) nur mittels Zweihandbedienung aus dem Gehäuse (1) entnehmbar ist.

13. Medizinisches Instrument nach Anspruch 11, **gekennzeichnet durch** einen zwischen wenigstens einer Wand (3) sowie einem Werkzeug (2) zusammenwirkenden Entriegelungsmechanismus (9).

14. Medizinisches Instrument nach Anspruch 13, **dadurch gekennzeichnet, daß** der Entriegelungsmechanismus (9) einen in einer Wand (3) gelagerten Entriegelungsknopf (10) aufweist.

15. Medizinisches Instrument nach Anspruch 14, **dadurch gekennzeichnet, daß** der Entriegelungsknopf (10) federnd in der Wand (3) gelagert ist, insbesondere auf einer in einer Ausnehmung (13) der Wand (3) gelagerten, freigeschnittenen Federzunge (14).

16. Medizinisches Instrument nach Anspruch 11, **gekennzeichnet durch** einen zwischen dem Basisteil (4) sowie einem Werkzeug (2) zusammenwirkenden Kopplungsmechanismus.

17. Medizinisches Instrument nach Anspruch 16, **dadurch gekennzeichnet, daß** der Kopplungsmechanismus ein im Basisteil (4) gelagertes Federelement (12) aufweist.

18. Medizinisches Instrument nach Anspruch 17, **dadurch gekennzeichnet, daß** in den Werkzeugen (2) Ausnehmungen (16) derart ausgebildet sind, daß das Federelement (12) in der Ruhestellung der Werkzeuge (2) in die Ausnehmungen (16) zweier parallel nebeneinander im Gehäuse (1) angeordneter Werkzeuge (2) eingreift.

19. Medizinisches Instrument nach Anspruch 17 oder 18, **dadurch gekennzeichnet, daß** pro parallel im Gehäuse (1) angeordnetem Werkzeugpaar ein Federelement (12) vorgesehen ist.

20. Medizinisches Instrument nach Anspruch 19, **dadurch gekennzeichnet, daß** mehrere im Gehäuse (1) angeordnete Federelemente (12) so miteinander gekoppelt verbunden sind, daß das Verlagern eines Federelements (12) aus der Ruhestellung die jeweils anderen Federelemente (12) blockiert.

21. Medizinisches Instrument nach mindestens einem der Ansprüche 17 bis 20, **dadurch gekennzeichnet, daß** in den Werkzeugen (2) Ausnehmungen (18) ausgebildet sind, in die das Federelement (12) in der Arbeitsstellung des Werkzeugs (2) eingreift.

22. Medizinisches Instrument nach mindestens einem der Ansprüche 14 bis 21, **dadurch gekennzeichnet, daß** der Entriegelungsknopf (10) einen Führungsstift (10a) aufweist, der in eine Führungsbahn (15) des entsprechenden Werkzeugs (2) eingreift.

23. Medizinisches Instrument nach mindestens einem der Ansprüche 13 bis 22, **dadurch gekennzeichnet, daß** am Basisteil (4) jeweils wenigstens ein in einen jeden Führungsschacht (8) hineinragender Vorsprung (25) ausgebildet ist, der in eine entsprechende Vertiefung (26) im jeweiligen Werkzeug (2) eingreift.

24. Medizinisches Instrument nach Anspruch 23, **dadurch gekennzeichnet, daß** am proximalen Ende der Vertiefung (26) eine Anlaufschräge (26a) ausgebildet ist.

25. Medizinisches Instrument nach mindestens einem der Ansprüche 1 bis 24, **dadurch gekennzeichnet, daß** die Ausfahrlänge des mindestens einen Werkzeugs (2) aus dem Gehäuse (1) durch einen Anschlag (15a) begrenzt ist.

26. Medizinisches Instrument nach Anspruch 25, **dadurch gekennzeichnet, daß** der Anschlag (15a) ein Teil der Führungsbahn (15) ist.

27. Medizinisches Instrument nach mindestens einem der Ansprüche 1 bis 26, **dadurch gekennzeichnet, daß** die Oberfläche des Gehäuses (1) glatt und frei von scharfen Kanten ausgebildet ist.

28. Medizinisches Instrument nach mindestens einem der Ansprüche 1 bis 27, **dadurch gekennzeichnet, daß** am Gehäuse (1) ein Lichtleiter und/oder ein flexibles Endoskop führend gelagert sind.

## Claims

1. Medical instrument for surgery, which can be introduced completely into the operating area for operating purposes together with one of the surgeon's hands, and which has a housing (1) and at least one tool (2) arranged in the housing (1), which tool (2) can be moved between a rest position arranged in the housing (1) and a working position extending out of the housing (1), at least one tool (2) being a gripper or cutter tool (20, 21) provided with two mutually adjustable jaw parts (22), in particular a needle gripper, and a finger ring (5) being arranged on the housing (1) for securing to the surgeon's hand, and the tool (20, 21) being partially retractable into the housing (1) for the purpose of fixing the jaw parts (22) in the closed position, **characterized in that** the finger ring (5) is connected to the housing (1) via a bridge (6) which is mounted pivotably on the housing (1).

2. Medical instrument according to Claim 1, **characterized in that** several tools (2) are arranged in the housing (1) so that they can be exchanged and so that they can be moved between the rest position and the working position.

3. Medical instrument according to Claim 1 or 2, **characterized in that** the at least one tool (2) is moved between the rest position and the working position by means of a translation movement.

4. Medical instrument according to Claim 1 or 2, **characterized in that** the at least one tool (2) is moved between the rest position and the working position by means of a rotation movement.

5. Medical instrument according to at least one of Claims 2 to 4, **characterized in that** only a single tool (2) can be moved out of the housing (1) and into the working position at any one time.

6. Medical instrument according to Claim 5, **characterized in that** the tool (2) moved into the working position fixes the other tools (2) inside the housing (1) in the rest position.

7. Medical instrument according to at least one of Claims 1 to 6, **characterized in that** the tool (2) moved into the working position is fixed securely in the working position.

8. Medical instrument according to at least one of Claims 1 to 7, **characterized in that** the two mutually adjustable jaw parts (22) are pretensioned in the opening direction of the jaw parts (22) via a spring element (24).

9. Medical instrument according to at least one of Claims 1 to 8, **characterized in that** the housing (1) consists of two walls (3) which are arranged substantially parallel to one another and are spaced apart from one another by a base part (4) arranged between the walls (3).

10. Medical instrument according to Claim 9, **characterized in that** the base part (4) is substantially T-shaped.

11. Medical instrument according to Claim 10, **characterized in that** the tools (2) are mounted between the walls (3) and the vertical arm (4a) of the T-shaped base part (4).

12. Medical instrument according to at least one of Claims 1 to 11, **characterized in that** the at least one tool (2) can be removed from the housing (1) only by using two hands.

13. Medical instrument according to Claim 11, **characterized by** an unlocking mechanism (9) acting between at least one wall (3) and one tool (2).

14. Medical instrument according to Claim 13, **characterized in that** the unlocking mechanism (9) has an unlocking knob (10) mounted in one wall (3).

15. Medical instrument according to Claim 14, **characterized in that** the unlocking knob (10) is mounted with a spring action in the wall (3), in particular on a free-cut spring tongue (14) which is mounted in a recess (13) in the wall (3).

16. Medical instrument according to Claim 11, **characterized by** a coupling mechanism acting between the base part (4) and a tool (2).

17. Medical instrument according to Claim 16, **characterized in that** the coupling mechanism has a spring element (12) mounted in the base part (4).

18. Medical instrument according to Claim 17, **characterized in that** recesses (16) are formed in the tools (2) in such a way that, in the rest position of the tools (2), the spring element (12) engages in the recesses (16) of two tools (2) arranged parallel to one another in the housing (1).

19. Medical instrument according to Claim 17 or 18, **characterized in that** one spring element (12) is provided for each pair of tools arranged in parallel in the housing (1).

20. Medical instrument according to Claim 19, **characterized in that** several spring elements (12) arranged in the housing (1) are connected to one another by coupling in such a way that the movement of one spring element (12) out of the rest position blocks the respective other spring elements (12).

21. Medical instrument according to at least one of Claims 17 to 20, **characterized in that** recesses (18) are formed in the tools (2), into which recesses (18) the spring element (12) engages in the working position of the tool (2).

22. Medical instrument according to at least one of Claims 14 to 21, **characterized in that** the unlocking knob (10) has a guide pin (10a) which engages in a guide track (15) of the corresponding tool (2).

23. Medical instrument according to at least one of Claims 13 to 22, **characterized in that** at least one projection (25) formed on the base part (4), and extending into each guide shaft (8), engages in a corresponding recess (26) in the respective tool (2).

24. Medical instrument according to Claim 23, **characterized in that** a run-up slope (26a) is formed at the proximal end of the recess (26).

25. Medical instrument according to at least one of Claims 1 to 24, **characterized in that** the distance by which the at least one tool (2) can be moved out of the housing (1) is limited by a stop (15a).

26. Medical instrument according to Claim 25, **characterized in that** the stop (15a) is a part of the guide track (15).

27. Medical instrument according to at least one of Claims 1 to 26, **characterized in that** the surface of the housing (1) is smooth and free of sharp edges.

28. Medical instrument according to at least one of Claims 1 to 27, **characterized in that** a light guide and/or a flexible endoscope is/are mounted on the housing (1).

## Revendications

1. Instrument médical pour la chirurgie, susceptible d'être introduit complètement dans le champ opératoire conjointement avec une main du chirurgien à des fins d'opération, et comportant un boîtier (1) et au moins un outil (2) agencé dans le boîtier (1) et mobile entre une position de repos agencée dans le boîtier (1) et une position de travail dépassant hors du boîtier (1), au moins un outil (2) étant un outil de préhension ou de coupe (20, 21), en particulier un préhenseur à aiguille, pourvu de deux parties de mâchoire (22) déplaçables l'une par rapport à l'autre, et un anneau à doigt (5) pour l'immobilisation sur la main du chirurgien étant agencé sur le boîtier (1), l'outil (20, 21) pouvant être rétracté partiellement dans le boîtier (1) pour fixer les parties de mâchoire (22) dans la position fermée, **caractérisé en ce que** l'anneau à doigt (5) est relié au boîtier (1) via une barrette (6) montée en pivotement sur le boîtier (1).

2. Instrument médical selon la revendication 1, **caractérisé en ce que** plusieurs outils (2) sont agencés dans le boîtier (1) en étant interchangeables et mobiles entre la position de repos et la position de travail.

3. Instrument médical selon l'une ou l'autre des revendications 1 et 2, **caractérisé en ce que** le déplacement dudit au moins un outil (2) entre la position de repos et la position de travail s'effectue par un mouvement de translation.

4. Instrument médical selon l'une ou l'autre des revendications 1 et 2, **caractérisé en ce que** le déplacement dudit au moins un outil (2) entre la position de repos et la position de travail s'effectue par un mouvement de rotation.

5. Instrument médical selon l'une au moins des revendications 2 à 4, **caractérisé en ce que** toujours un seul outil (2) est mobile hors du boîtier (1) jusque dans la position de travail.

6. Instrument médical selon la revendication 5, **caractérisé en ce que** l'outil (2) déplacé dans la position de travail fixe les autres outils (2) à l'intérieur du boîtier (1) dans la position de repos.

7. Instrument médical selon l'une au moins des revendications 1 à 6, **caractérisé en ce que** l'outil (2) déplacé dans la position de travail est fixé de façon stationnaire dans la position de travail.

8. Instrument médical selon l'une au moins des revendications 1 à 7, **caractérisé en ce que** les deux parties de mâchoire (22) déplaçables l'une par rapport à l'autre sont précontraintes en direction d'ouverture des parties de mâchoire (22) par un élément élastique (24).

9. Instrument médical selon l'une au moins des revendications 1 à 8, **caractérisé en ce que** le boîtier (1) est constitué par deux parois (3) agencées sensiblement parallèlement l'une à l'autre qui sont écartées l'une de l'autre via un élément de base (4) agencé entre les parois (3).

10. Instrument médical selon la revendication 9, **caractérisé en ce que** l'élément de base (4) est réalisé sensiblement en forme de T.

11. Instrument médical selon la revendication 10, **caractérisé en ce que** les outils (2) sont montés entre les parois (3) et la branche verticale (4a) de l'élément de base (4) en forme de T.

12. Instrument médical selon l'une au moins des revendications 1 à 11, **caractérisé en ce que** ledit au moins un outil (2) est susceptible d'être enlevé hors du boîtier (1) uniquement par une manipulation à l'aide des deux mains.

13. Instrument médical selon la revendication 11, **caractérisé par** un mécanisme de déverrouillage (9) coopérant entre au moins une paroi (3) et un outil (2).

14. Instrument médical selon la revendication 13, **caractérisé en ce que** le mécanisme de déverrouillage (9) comprend un bouton de déverrouillage (10) monté dans une paroi (3).

15. Instrument médical selon la revendication 14, **caractérisé en ce que** le bouton de déverrouillage (10) est monté élastiquement dans la paroi (3), en particulier sur une languette élastique (14) dégagée par découpe montée en particulier dans un évidement (13) de la paroi (3).

16. Instrument médical selon la revendication 11, **caractérisé par** un mécanisme d'accouplement coopérant entre l'élément de base (4) et un outil (2).

17. Instrument médical selon la revendication 16, **caractérisé en ce que** le mécanisme d'accouplement comprend un élément élastique (12) monté dans l'élément de base (4).

18. Instrument médical selon la revendication 17, **caractérisé en ce que** des évidements (16) sont ménagés dans les outils (2) de telle sorte que l'élément élastique (12) s'engage, en position de repos des outils (2), dans les évidements (16) de deux outils (2) agencés parallèlement l'un à côté de l'autre dans le boîtier (1).

19. Instrument médical selon l'une ou l'autre des revendications 17 et 18, **caractérisé en ce qu'**il est prévu un élément élastique (12) pour chaque paire d'outils agencés parallèlement dans le boîtier (1).

20. Instrument médical selon la revendication 19, **caractérisé en ce que** plusieurs éléments élastiques (12) agencés dans le boîtier (1) sont mutuellement reliés en étant accouplés de telle sorte que le déplacement d'un élément élastique (12) hors de la position de repos bloque les autres éléments élastiques respectifs (12).

21. Instrument médical selon l'une au moins des revendications 17 à 20, **caractérisé en ce que** des évidements (18) sont ménagés dans les outils (2), dans lesquels s'engage l'élément élastique (12) dans la position de travail de l'outil (2).

22. Instrument médical selon l'une au moins des revendications 14 à 21, **caractérisé en ce que** le bouton de déverrouillage (10) comprend une tige de guidage (10a) qui s'engage dans une voie de guidage (15) de l'outil correspondant (2).

23. Instrument médical selon l'une au moins des revendications 13 à 22, **caractérisé en ce qu'**au moins une saillie respective (25) pénétrant chacune dans une lumière de guidage respective (8) est réalisée sur l'élément de base (4), saillie qui s'engage dans un renfoncement correspondant (26) dans l'outil respectif (2).

24. Instrument médical selon la revendication 23, **caractérisé en ce qu'**une pente de montée (26a) est réalisée à l'extrémité proximale du renfoncement (26).

25. Instrument médical selon l'une au moins des revendications 1 à 24, **caractérisé en ce que** la longueur de déploiement dudit au moins un outil (2) hors du boîtier (1) est limitée par une butée (15a).

26. Instrument médical selon la revendication 25, **caractérisé en ce que** la butée (15a) fait partie de la voie de guidage (15).

27. Instrument médical selon l'une au moins des revendications 1 à 26, **caractérisé en ce que** la surface du boîtier (1) est lisse et dépourvue d'arêtes vives.

28. Instrument médical selon l'une au moins des revendications 1 à 27, **caractérisé en ce qu'**un guide de lumière et/ou un endoscope flexible sont montés avec guidage sur le boîtier (1).
